# EUROPEAN PATENT SPECIFICATION

(11) **EP 4 486 942 B1**
(45) Date of publication and mention of the grant of the patent: **26.11.2025**
(21) Application number: 23712161.1
(22) Date of filing: 02.03.2023
(51) Int. Cl.: D06M 13/00, C07D 307/50, B29B 17/02, C08J 11/06, C08J 11/08, C07D 307/48

(54) **METHOD OF EXTRACTING 5-CHLOROMETHYLFURFURAL WITH AN ORGANIC SOLVENT FROM CELLULOSIC FIBERS AND MAN-MADE NON-CELLULOSIC FIBERS HYDROLYSED TOGETHER WITH HYDROCHLORIC ACID**
VERFAHREN ZUR EXTRAKTION MIT EINEM ORGANISCHEN LÖSUNGSMITTEL VON 5-CHLOROMETHYLFURFURAL, HERGESTELLT DURCH DIE HYDROLYSE VON ZELLULOSEHALTIGEN FASERN SUZAMMEN MIT KÜNSTLICHEN NICHT-ZELLULOSEHALTIGEN FASERN
PROCÉDÉ D'EXTRACTION PAR UN SOLVANT ORGANIQUE DU 5-CHLORAMETHYLFURURAL PRODUIT PAR L'HYDROLYSE AVEC DE L'ACIDE CHLORHYDRIQUE DE FIBRES DE CELLULOSE AVEC DES FIBRES SYNTHETIQUES NON CELLULOSIQUES MÉLANGÉES.

(30) Priority: 04.03.2022 EP 22160327
(43) Date of publication of application: 08.01.2025
(73) Proprietor: Avantium Knowledge Centre B.V., 1014 BV Amsterdam (NL)
(72) Inventor: BUENO MORON, Jorge, 1014 BV Amsterdam (NL); GRUTER, Gerardus Johannes Maria, 1014 BV Amsterdam (NL); VAN KLINK, Gerardus Petrus Maria, 1014 BV Amsterdam (NL)
(74) Representative: Avantium Intellectual Property
(86) International application number: PCT/EP2023/055303
(87) International publication number: WO 2023/166122

(56) References cited:
- EP-A1- 0 079 206
- EP-A1- 2 931 906
- WO-A1-2014/066746
- WO-A1-2016/025674
- WO-A1-2016/094878
- WO-A1-2019/140245
- WO-A1-2019/149843
- WO-A1-2019/149853
- WO-A1-2021/066695
- WO-A1-2022/146219
- WO-A2-2014/062303
- CN-A- 105 801 532
- CN-A- 112 574 143
- JP-A- 2015 172 020
- US-A1- 2009 234 142
- MARK MASCAL ET AL: "Dramatic Advancements in the Saccharide to 5-(Chloromethyl)furfural Conversion Reaction", CHEMSUSCHEM, vol. 2, no. 9, 21 September 2009 (2009-09-21), DE, pages 859 - 861, XP055259112, ISSN: 1864-5631, DOI: 10.1002/cssc.200900136
- ONKARAPPA SHARATH BANDIBAIRANAHALLI ET AL: "High-Yielding Synthesis of 5-(alkoxymethyl)furfurals from Biomass-Derived 5-(halomethyl)furfural (X=Cl, Br)", CHEMISTRYSELECT, vol. 4, no. 19, 20 May 2019 (2019-05-20), DE, pages 5540 - 5543, XP055946915, ISSN: 2365-6549, Retrieved from the Internet <URL:https://onlinelibrary.wiley.com/doi/full-xml/10.1002/slct.201900279> DOI: 10.1002/slct.201900279
- RUUTH EDVIN: "Chemical recycling of cotton-based waste textiles by sulfuric acid hydrolysis", MASTER THESIS LUND UNIVERSITY, 1 June 2020 (2020-06-01), pages 1 - 27, XP055773160, Retrieved from the Internet <URL:http://lup.lub.lu.se/luur/download?func=downloadFile&recordOId=9018398&fileOId=9018404> [retrieved on 20210208]
- JEIHANIPOUR AZAM ET AL: "Acid Hydrolysis of Cellulose-based Waste Textiles", THE 7TH INTERNATIONAL CHEMICAL ENGINEERING CONGRESS & EXHINITION KISH, IRAN 21-24 NOV. 2011, 21 November 2011 (2011-11-21), pages 1 - 5, XP055946902
- YOUSEF SAMY ET AL: "A new strategy for using textile waste as a sustainable source of recovered cotton", RESOURCES, CONSERVATION AND RECYCLING, vol. 145, 19 March 2019 (2019-03-19), pages 359 - 369, XP085657087, ISSN: 0921-3449, DOI: 10.1016/J.RESCONREC.2019.02.031
- NITEE KUMARI ET AL: "Synthesis of 5-Bromomethylfurfural from Cellulose as a Potential Intermediate for Biofuel", EUROPEAN JOURNAL OF ORGANIC CHEMISTRY, vol. 2011, no. 7, 21 January 2011 (2011-01-21), pages 1266 - 1270, XP055099148, ISSN: 1434-193X, DOI: 10.1002/ejoc.201001539
- ROBINSON EMILY GRACE: "Textile recycling via ionic liquids Article Info", 1 September 2020 (2020-09-01), XP055946905, Retrieved from the Internet <URL:https://digital.lib.washington.edu/researchworks/bitstream/handle/1773/46257/pp62-65_20200901_Robinson_EG_P2.pdf?sequence=4> [retrieved on 20220727], DOI: 10.6069/6F1V-JC85

## Description

### Introduction

The present invention relates to a process for treating fabric material, which fabric material comprises cellulosic fibre material (e.g. from cotton, flax, jute, ramie, hemp, Pina and Abaca) and non-cellulosic, man-made polymer fibre material, such as polyester and/or polyolefins (polyethylene or polypropylene), polyamide fibre material or elastane fibre material. The treatment relates to hydrolysing at least part of the cellulose fibre material (e.g. cotton) that is present in the mixed fabric material with concentrated hydrochloric acid, and converting the hydrolysed cellulose into 5-(chloromethyl)furfural (5-CMF). So-obtained 5-CMF may be further processed e.g. into 5-(hydroxymethyl)furfural, 5-alkoxymethylfurfural, 5-(acetoxymethyl)furfural, methylfurfural or isolated as 5-CMF.

### Background of the invention

Patent application WO2019/149843 discloses a process that obtains a chemical building block from solid lignocellulosic material such as woodchips. Such lignocellulosic material contains as main fractions hemicellulose, cellulose and lignin. The process of the referred patent application subjects the substantially dried lignocellulosic material first to hydrochloric acid in a concentration of 15 to 40% at room temperature, to hydrolyse and remove from the lignocellulosic material and thus from the reactor the hemicellulose. Subsequently, the remaining material is subjected to hydrochloric acid of 40-51% strength at room temperature, to hydrolyse and remove from the reactor the cellulose fraction, leaving behind the solid lignin residue in the reactor. The process continues by subjecting the hydrolysate of cellulose obtained (which next to the carbohydrates like glucose and glucose oligomers) still contains hydrochloric acid) to heating, to yield 5-(chloromethyl)furfural (5-CMF). The so-obtained 5-CMF can be extracted with a solvent. 5-CMF can be converted into various other chemicals, and hence is a convenient chemical building block. Similar to the above reference, WO2014/066746 discloses a process for producing 5-(chloromethyl)-furfural by acid-catalysed conversion of biomass.

Cellulose is also present as cellulosic fibres in fabrics such as textile for e.g. clothing. Cellulosic fibre material such as cotton has many uses, e.g. in fabric for textile for use in clothing. Such clothing has a finite life, e.g. due to wear. The end-of-life of cotton-containing clothing, optionally after recycling to suit other uses, often means ending up in land-fill or waste incineration with or without energy recovery. More and more, land-fill as a destination of clothing after use is seen as undesired, and recycling is investigated. Recycling high-value cotton fibre into new cellulose-containing fibre for use in textile for clothing is often not possible, largely due to cotton (and other cellulosic fibres) being used in textile mixed with other fibres (in varying amounts), such as polyester, to give the textile the desired properties (e.g. strength, cost-effectiveness, ease of manufacture and others). Textiles containing such mixture of cellulosic and non-cellulosic fibres are hitherto hard or impossible to recycle into a valuable product. Even clothing that is stated to be 100% cotton, often has stitching of polyester yarn, and this also is a "mixed fibre textile". Apart from ending up in land-fill or incineration facilities, such mixed fibre textiles are used to make e.g. rags. That is re-using as textile, but it is down-cycling rather than recycling, as rags (e.g. for industrial cleaning) are a low-value product, and there is a limited use for such products.

Cellulosic fibre is also used as an ingredient in medical wipes, e.g. for cleaning and/or disinfection, which find use in e.g. hospitals, care institutions, and centres for medical services such as vaccination and testing for infectious diseases. To minimize risk of contamination or infection for patients and personnel, such medical wipes are used abundantly, and have a single use. Clearly, this leads to a large amount of waste material of such textile. All sorts of material are used for medical wipes, but a large selection contains cotton and a non-cellulosic, man-made fibre such as polyester. Re-using is virtually impossible or undesired, and the material is usually incinerated after being discarded.

Hence, there is a desire for being able to turn discarded clothing or fabric such as medical wipes made of fabric that contains both cotton or other cellulosic fibre as well as a non-cellulosic fibre such as polyester (typically PET) into more valuable products than into rags, to serve as an alternative to being incinerated or used as land-fill. Likewise, there is a need for processes that allow chemical building blocks to be made from sources which are not fully based on fossil-carbon sources.

### Summary of the invention

It has now been found objective that the above may be achieved, at least in part, by a process for treating mixed fabric material, said mixed fabric material comprising cellulosic fibre material in an amount of 30 - 99% by weight and comprising 1 - 70% by weight of one or more of man-made polyester fibre material, polyethylene fibre material, polypropylene fibre material, polyamide fibre material, or elastane fibre material, said process comprising:
a. providing in a reactor:
   - said mixed fabric material, comprising cellulosic fibre material in an amount of 30 - 99% by weight and comprising 1 - 70% by weight of one or more of man-made polyester fibre material, polyethylene fibre material, polypropylene fibre material, polyamide fibre material or elastane fibre material,
   - an aqueous solution of hydrochloric acid, the aqueous solution having a concentration of hydrochloric acid of 30% - 45% (by weight),
   - an organic extraction solvent, and wherein the organic extraction solvent is chosen such that at 20°C less than 1 g of extraction solvent dissolves in 100 ml of the aqueous HCI solution, and wherein the organic extraction solvent is further chosen such that at 20°C at least 10 g of 5-(chloromethyl)furfural (5-CMF) can be dissolved in 100 ml of the organic extraction solvent,
b. mixing the contents of the reactor at a temperature of between 40°C and 110°C for at least 30 minutes and wherein the aqueous solution and the organic extraction solvent are present in the reactor in a ratio aqueous solution : organic extraction solvent of between 1 : 0.5 and 1: 10,
c. separating the organic extraction solvent containing 5-(chloromethyl)furfural (5-CMF) from the aqueous solution comprising hydrochloric acid.

In an alternative way, relying on the same principle, the above may be achieved, at least in part, by a process for treating mixed fabric material, said mixed fabric material comprising cellulosic fibre material in an amount of 30 - 99% by weight and comprising 1 - 70% by weight of one or more of man-made polyester fibre material, polyethylene fibre material, polypropylene fibre material, polyamide fibre material or elastane fibre material, said process comprising:
a. providing in a reactor:
   - said mixed fabric material, comprising cellulosic fibre material in an amount of 30 - 99% by weight and comprising 1 - 70% by weight of one or more of man-made polyester fibre material, polyethylene fibre material, polypropylene fibre material, polyamide fibre material or elastane fibre material,
   - an aqueous solution of hydrochloric acid, the aqueous solution having a concentration of hydrochloric acid of 36% - 51% (by weight),
   for a time of at least 30 minutes and at a temperature of below 40°C to at least partly hydrolyse the cellulose in the cellulose fibre material, to yield an aqueous hydrolysate and solid fibrous residue,
b. removing from the product of a. the solid fibrous residue,
c. mixing the aqueous hydrolysate obtained by b. with an organic extraction solvent at a temperature of between 50°C and 110°C for at least 30 minutes and wherein the aqueous solution and the organic extraction solvent are present in the reactor in a ratio aqueous solution : organic extraction solvent of between 1 : 0.5 and 1: 10, and wherein the organic extraction solvent is chosen such that at 20°C less than 1 g of extraction solvent dissolves in 100 ml of the aqueous HCI solution and wherein the organic extraction solvent is further chosen such that at 20°C at least 10 g of 5-(chloromethyl)furfural (5-CMF) can be dissolved in 100 ml of the organic extraction solvent,
d. separating the organic extraction solvent containing 5-(chloromethyl)furfural (5-CMF) from the aqueous solution comprising hydrochloric acid.

### Detailed description of the invention

The above two processes are two embodiments of the same invention, which resides in the fact that cellulose in a mix of fibres containing both cellulose and man-made polyester, polyolefins, polyamide fibre and elastane fibre, may be used as a source to yield 5-(chloromethyl)furfural (5-CMF) by hydrolysis of the cellulose followed by conversion of glucose/glucose oligomers into 5-CMF by the action of hydrochloric acid, extraction of the 5-CMF with an organic extractant, whilst the man-made polyester fibres, polyolefin fibres, polyamide fibres and elastane fibre are left largely or completely physically intact. The man-made polyester, polyolefins, polyamide and elastane fibre are resistant to the action of hydrochloric acid in this process.

In the processes according to the invention, the stage in which the hydrochloric acid is reacting with the cellulose is preferably conducted for a period during which at least 50% (by weight) of the cellulosic fibre material is hydrolysed, preferably at least 70% is hydrolysed. The actual time depends e.g. on the temperature, nature of the fibres, and other process parameters, and can easily be determined by the skilled person. Typically, such takes at least 30 minutes, but may be carried out longer, e.g. at least 1 hour, or preferably 2 hours or more. This stage, however, should preferably not last more than 12 hours, more preferably not more than 10 hours, and even more preferably not more than 6 hours.

It will be clear to the skilled person that the mixing in stage b. (1^{st} embodiment) and stage c. (2^{nd} embodiment) should be fairly vigorous, as two liquids are present which are largely immiscible, and the 5-CMF formed in the aqueous phase needs to "transfer" to the organic extractant.

As set out above, so far mixed fibre blends of e.g. cotton with one or more of polyester, polyolefins, polyamide, or elastane after usage for what the fibre blends were designed for (clothing, cleaning, protection, domestic use like in curtains, etcetera) not be put to much other use than for making rags or for incineration. It was surprisingly found that by the above process, a mixed fibre blend of cotton and one or more of polyester fibres (e.g. PET), polyolefin fibres, polyamide fibres, or elastane fibres was suitable as a source to produce a useful chemical intermediate compound 5-(chloromethyl)furfural (5-CMF). Even more surprising and beneficial, the process according to the present invention allows for the residual man-made polymer material, such as PET, to be mechanically or chemically recycled to recycled PET or rPET.

The 5-CMF formed by the action of hydrochloric acid on the cellulose is dissolved, (or mixed, if at sufficient high temperature for the 5-CMF being liquid) and preferentially is dissolved predominantly in the organic extraction liquid. The polyester, polyolefin fibres, polyamide fibres or elastane fibres are largely unaffected by the process, and can be physically removed from the resulting liquids: aqueous hydrochloric acid, optionally with 5-CMF or small saccharides dissolved in such and organic extraction liquid with 5-CMF dissolved in such. Hence, it is preferred in the process of the invention that after stage b. (for the 1^{st} embodiment mentioned) any solid matter, including solid fibre material residue, is removed from the liquid phase. The liquid phase here comprises the aqueous phase comprising hydrochloric acid, and the organic extraction liquid comprising 5-CMF. The non-liquid matter that is removed by such step comprises one or more of polyester fibre material, polyethylene fibre material, polypropylene fibre material, polyamide fibre material or elastane fibre material.

The 5-CMF that is formed by the action of hydrochloric acid on the solubilized / hydrolysed cellulose does dissolve (and or mix, as the melting point of 5-CMGF is about 34°C) in the aqueous hydrochloric acid, but is difficult to isolate therefrom and it may also decompose in the strong acid medium. If an organic extraction solvent is present during the 5-CMF production (or after the conversion of hydrolyzed cellulose to 5-CMF in the alternative embodiment), in which the 5-CMF dissolves easier than in the aqueous hydrochloric acid, and if the organic extraction solvent is selected such that it does not mix well with water, the 5-CMF formed can be extracted from the aqueous hydrochloric acid phase relatively easily during its formation. There is a range of potentially suitable organic extraction solvents for this process. A high distribution coefficient (the ratio of the solubility of 5-CMF in the organic phase and the aqueous HCI phase) is desired to maximize 5-CMF extraction and to minimize extraction solvent volume. The solvent should be selected on the basis of price, extraction coefficient, boiling point (preferably above 80°C) stability, etc. Hence, in the process according to the present invention it is preferred that the organic extraction solvent comprises: diisopropyl ether, ethyl acetate, pentane, hexane, heptane, octane, decane, dodecane, cyclohexane, benzene, chlorobenzene, toluene, xylene, chloroform, 1,2-dichloroethane, carbon tetrachloride, trichloromethane (chloroform), and mixtures thereof. A preferred class of extraction solvents for this purpose are halogenated aromatic solvents: aromatic compound with one or more halogen atoms present. Preferred halogenated aromatic solvents in this context comprise chlorobenzene, dichlorobenzene (*o-, m*- and *p*- included), trichlorobenzene (1,2,3-, 1,2,4-, 1,3,5- included), fluorobenzene, difluorobenzene (*o-, m*- and *p*- included), trifluorobenzene (1,2,3-, 1,2,4-, 1,3,5- included) and mixed halobenzene compounds, for example C₆H₄FCl (*o-, m*- and *p*-). Most preferred are organic extraction solvents that comprises toluene, chlorobenzene, dichlorobenzene, fluorobenzene, difluorobenzene, 1,2-dichloroethane, or mixtures thereof.

In the present process, a key component is the fabric fibre material containing cellulose. The most abundantly used materials of that kind are cotton and flax, jute, ramie, hemp, Pina and Abaca. Hence, in the present invention it is preferred that the cellulosic fibre comprises one or more of cotton, flax, jute, ramie, hemp, Pina and Abaca, with cotton being most preferred as cellulosic fibre material in the present invention. Clearly, in order to yield attractive yields of 5-CMF it is desired that the mixed fibre material contains a substantial amount of cellulose. Hence, in the present invention it is preferred that the mixed fabric material comprises cellulosic fibre material in an amount of 40 - 98% by weight, preferably 50-98% by weight. However, commercial mixed fibre materials contain fibres like polyester, polyamide fibre, elastane fibres or polyethylene or polypropylene in functional amounts. In this connection, it is preferred in the present invention that the mixed fabric material comprises 2 - 40% by weight of one or more of man-made polyester fibre material, polyethylene fibre material, polypropylene fibre material, preferably 2 -30% by weight. A most typical man-made polyester fibre is preferably PET, which is thus preferred as polyester in the present invention. The mixed fabric material can comprise woven material, non-woven material, braided material or mixtures thereof. An example of woven mixed textile material are textiles such as are used for e.g. the manufacture of clothing, or textiles for use for e.g. curtains and upholstery. An example of non-woven mixed fabric materials are e.g. those used for making cleaning and disinfection wipes.

The mixture of fabric material entered into the reactor preferably has a moisture content of below 10% by weight, more preferably below 7% by weight, even more preferably below 5% by weight, as e.g. otherwise the hydrochloric acid is too much diluted.

The conversion of cellulose to 5-CMF by aqueous hydrochloric acid and its extraction in an organic extraction liquid can be conducted in a few hours. Hence, in the process according to the present invention it is preferred that in stage b. (1^{st} embodiment) and stage c (2^{nd} embodiment), the mixing of the contents of the reactor is conducted for a time of between 0.5 hours and 12 hours, preferably between 1 and 10 hours. It is preferred in the process of the present invention that the amount of mixed fabric material : aqueous solution of hydrochloric acid in a. is between 1 : 200 and 1 : 5, preferably between 1 : 100 and 1 : 20.

The 5-CMF obtained in the organic extraction liquid can be isolated or can be processed further in other chemical useful components, such as to alkoxymethyl furfural, e.g. methoxymethyl furfural (MMF) and ethoxymethyl furfural (EMF), to HMF, HMF acetate (AcMF) or to methylfurfural (MF). This can be done with processes which are known as such by reacting 5-CMF with methanol, ethanol, water, acetic acid or hydrogen, respectively. Hence, in the present invention it may be preferred that the organic extraction solvent comprising 5-(chloromethyl)furfural (5-CMF) obtained by stage c. (1^{st} embodiment) or stage d. (2^{nd} embodiment) is mixed with liquid C1-C4-alkanol in a ratio CMF : alkanol of between 1 : 1 and 1 : 25 and subjected to a temperature of between 10 and 40°C to convert at least part of the 5-(chloromethyl)furfural (5-CMF) into 5-RMF (R being the groups as set out above). Such processes are e.g. known from S. Bandibairanahalli Onkarappa, Chemistry Select, 2019, 4, 5540-5543. In particular MMF and EMF are desired products, the first as an intermediate to produce furan dicarboxylic acid, and the second as component for fuel additives. Hence, in such conversion it is preferred that the liquid C1-C4-alkanol comprises methanol or ethanol, and preferably is methanol. In a similar way, HMF can be obtained by addition of water; MF can be obtained by CMF hydrogenation. In all these 5-CMF conversions, a catalyst can be added to accelerate the CMF conversion reaction.

Alternatively, the 5-(chloromethyl)furfural (5-CMF) obtained in solution in stage c. (1^{st} embodiment) or stage d. (2^{nd} embodiment) can be isolated from the organic extraction solvent . By evaporation of the solvent followed by subsequent conversion, optionally after distillation or re-crystallization of the CMF. Conversion of CMF to RMF, HMF, AMF or MF can be performed in neat alcohol (to RMF), water (to HMF) or acetic acid (to AMF) or in the presence of an additional suitable solvent.

### EXAMPLES

### Example 1

### Materials and equipment

The reaction was performed in small batch reactor tubes (volume: 9 ml): Ace glass high pressure tubes purchased in Sigma Aldrich (product code: 8648-17), and were equipped with magnetic stirrers. These were heated in alumina heating blocks custom made.
Aqueous phase: 37%wt hydrochloric acid
Organic phase: Chlorobenzene (99.5%, Acros Organics, examples 1-6), fluorobenzene (examples 5, 6) Fabric:
   - pieces of commercial "jeans", ~98%wt. cotton (label), Analytical results (Celignis Biomass Laboratory), average of triplo analysis, sugar 95.34%, total lignin 2.05%, ash 0.60%.
   - PET textile fibre: overlock PET yarn produced by Diamond Spun Garens.
   - pieces of commercial (yellow) polycotton sweater (80%wt cotton, 20%wt polyester (label).
   - pieces of commercial socks (70%wt cotton, 28%wt polyester, 2%wt Spandex (label)).

### Procedure

The reactor was loaded with the desired amount of cotton-containing jeans fabric (10-25 mg, the amount differed due to difficulty cutting small pieces of fabric), and PET fibers (10-25 mg, range for the same reason as for the pieces of jeans) were added to that, and further 1 mL of hydrochloric acid was added. Lastly the organic phase (chlorobenzene, 3 mL) was added on top of it. The reactor was closed and placed inside the alumina block already pre-heated at the desired temperature, under mixing. As to the desired temperature: reactions were carried out at 80, 90 and 100 °C to study the influence of the temperature. After 1 hour of reaction time, the vials were taken out of the block, cooled down in an ice bath for 15 minutes. The resulting aqueous liquid layer was darkly coloured, and contained fibrous material. The layer of organic liquid on top appeared clear and colourless. The organic layer (in this case on top of the liquid mixture) was removed . Thereafter, a further 3mL of organic phase was added. Subsequently, the reaction tube was put back in the pre-heated block at the desired temperature for another hour. This was repeated until samples were obtained after 1 hour, 2 hours, 3 hours and 4 hours of reaction. This was performed for three different temperatures, and each reaction was carried out three times. This gave 3x3x4 = 36 samples.

The samples were analysed by GC as to the content of 5-(chloromethyl)furfural (5-CMF) without further work-up, by using a 1 mL sample of the organic fraction with added thereto an external standard (0.1 mL dioxane in 0.9 mL toluene).

### Results

From the GC results the molar yield of 5-(chloromethyl)furfural (5-CMF) on cotton was calculated. The results are set out in table 1 below.

**Table 1: Molar yield of 5-CMF on cotton of example 1**

| sample nr | 1 hour | 2 hours | 3 hours | 4 hours | Cumulative after 4 hours | temperature (°C) |
|---|---|---|---|---|---|---|
| R#01 | 14.4 | 16.6 | 13.3 | 0.0 | 44.3 | 80 |
| R#02 | 18.8 | 17.2 | 0.0 | 0.0 | 36.1 | 80 |
| R#03 | 11.4 | 9.7 | 9.6 | 8.3 | 38.9 | 80 |
| R#04 | 21.0 | 15.2 | 11.1 | 0.0 | 47.3 | 90 |
| R#05 | 31.3 | 20.0 | 17.4 | 0.0 | 68.7 | 90 |
| R#06 | 20.6 | 14.5 | 11.1 | 0.0 | 46.1 | 90 |
| R#07 | 23.8 | 14.4 | 9.0 | 0.0 | 47.2 | 100 |
| R#08 | 26.5 | 10.6 | 0.0 | 0.0 | 37.1 | 100 |
| R#09 | 20.0 | 19.1 | 0.0 | 0.0 | 48.1 | 100 |

The results show a cumulative molar yield of 5-(chloromethyl)furfural (5-CMF) on cotton of between about 40 and 70%, and PET fibres which appeared visually intact. The PET fibers were dried and weighed which indicated full recovery of the plastic material. Subsequently, glycolysis (with ethylene glycol) showed that the material was indeed PET and could be recycled to near quantitatively to BHET (bis(2-hydroxyethyl)terephthalate): from the 10-25 mg PET, 12-30 mg BHET was obtained (qualification and quantification by HPLC using dimethylterephthalate as an external standard.

### Example 2

Example 1 was repeated, except that now 40-50 mg of jeans fabric was used, and 2 mL of hydrochloric acid solution (37% wt) was used. The amount of chlorobenzene added for extracting the samples was the same as in example 1 (3mL for each extraction). Timing for extracting the samples was after 2 hours, after 4 hours, and after 6 hours. Temperatures selected for the trails were 80 and 90°C. Like for example 1, trials were carried out in triplo. Analysis was done in the same way. The results are set out in table 2.

**Table 2: Cumulative molar yield of 5-CMF on fabric of example 2**

| sample nr | 6 hours | temperature (°C) |
|---|---|---|
| R#01 | 51.3 | 80 |
| R#02 | 37.3 | 80 |
| R#03 | 51.1 | 80 |
| R#04 | 40.0 | 90 |
| R#05 | 35.3 | 90 |
| R#06 | 40.4 | 90 |

### Example 3

Example 1 was repeated, except that now the jeans fabric was stirred at room temperature overnight with 2 mL of 42 wt% hydrochloric acid solution and 3 mL of chlorobenzene. After 16 h, the reactors were placed in the pre-heated alumina blocks at 80 °C. The amount of chlorobenzene added for extracting the samples was the same as in example 1 (3mL for extraction after 1 and 2 h, 5 mL for extraction after 4, 6, and 8 h). Timing for extracting the samples was after stirring overnight, and after 1 hour, 2 hours, after 4 hours, after 6 hours, and after 8 hours at 80 °C. Like for example 1, trials were carried out in triplo. Analysis was done in the same way. The results are set out in table 3.

**Table 3: Molar yield of 5-CMF on fabric of example 3**

| Sample nr | 1 h | 2 h | 4 h | 6 h | 8 h | total |
|---|---|---|---|---|---|---|
| R#01 | 23.1 | 15.5 | 12.4 | 8.1 | 4.3 | 63.4 |
| R#02 | 24.5 | 10.1 | 7.2 | 4.9 | 2.4 | 49.2 |
| R#03 | 39.0 | 7.9 | 7.3 | 2.1 | 2.4 | 58.5 |

### Example 4

Example 3 was repeated, except that now 3 vials with jeans fabric were stirred overnight with 2 mL of 42 wt% hydrochloric acid solution and 3 mL of chlorobenzene at room temperature while an additional vial was stirred overnight at 30 °C. In addition, the extraction times were shortened. After 16 h, the reactors were placed in the pre-heated alumina blocks at 80 °C. The amount of chlorobenzene added for extracting the samples was the same as in example 1 (3mL for each extraction). For the first hour, every 20 mins reactions time the reactions mixture was extracted in the usual manner. After 1 h, the procedure was executed as described above until 5 hours of total reaction time. The results are set out in table 4.

**Table 4: Molar yield of 5-CMF on jeans fabric of example 4. R#04 was heated overnight at 30 °C.**

| Sample nr | 20 min | 40 min | 1h | 2 h | 3 h | 4 h | 5 h | Total |
|---|---|---|---|---|---|---|---|---|
| R#01 | 12.3 | 13.1 | 8.5 | 4.8 | 4.1 | 2.3 | 0 | 45.1 |
| R#02 | 19.2 | 14.2 | 8.0 | 6.4 | 3.0 | 2.5 | 1.7 | 54.9 |
| R#03 | 17.5 | 16.2 | 8.6 | 9.2 | 3.8 | 1.7 | 1.3 | 58.2 |
| R#04 | 19.8 | 15.9 | 7.9 | 7.9 | 3.0 | 2.2 | 1.4 | 58.1 |

### Example 5

Example 1 was repeated, except that now 50 mg of a polycotton sweater fabric and 1 mL of hydrochloric acid solution (37% wt) was used. The amount of chlorobenzene or fluorobenzene was 3 mL. The reaction was run at 90 °C for 3 h. Like for example 1, trials were carried out in triplo. Analysis was done in the same way. The results are set out in table 5.

**Table 5: Molar yield of 5-CMF on mixed polycotton fabric (sweater) of example 5.**

| Sample nr | Solvent | Reaction Temperature (°C) | Reaction Time (h) | Molar Yield CMF |
|---|---|---|---|---|
| R#01 | Chlorobenzene | 90 | 3 | 51.3 |
| R#02 | Chlorobenzene | 90 | 3 | 52.4 |
| R#03 | Chlorobenzene | 90 | 3 | 52.0 |
| R#04 | Fluorobenzene | 90 | 3 | 44.7 |
| R#05 | Fluorobenzene | 90 | 3 | 44.4 |
| R#06 | Fluorobenzene | 90 | 3 | 43.9 |

### Example 6

Example 5 was repeated, except that now 55 mg of socks fabric and 1 mL of hydrochloric acid solution (37% wt) was used. The amount of chlorobenzene or fluorobenzene was 3 mL. The reaction was run at 90 °C for 3 h. Like for example 1, trials were carried out in triplo. Analysis was done in the same way. The results are set out in table 6.

**Table 6: Molar yield of 5-CMF on mixed polycotton fabric (socks) of example 6.**

| Sample nr | Solvent | Reaction Temperature (°C) | Reaction Time (h) | Molar Yield CMF |
|---|---|---|---|---|
| R#01 | Chlorobenzene | 90 | 3 | 30.0 |
| R#02 | Chlorobenzene | 90 | 3 | 33.3 |
| R#03 | Chlorobenzene | 90 | 3 | 33.8 |
| R#04 | Fluorobenzene | 90 | 3 | 37.4 |
| R#05 | Fluorobenzene | 90 | 3 | 34.5 |
| R#06 | Fluorobenzene | 90 | 3 | 30.9 |

## Claims

1. Process for treating mixed fabric material, said mixed fabric material comprising cellulosic fibre material in an amount of 30 - 99% by weight and comprising 1 - 70% by weight of one or more of man-made polyester fibre material, polyethylene fibre material, polypropylene fibre material, polyamide fibre material, or elastane fibre material, said process comprising:
a. providing in a reactor:
- said mixed fabric material, comprising cellulosic fibre material in an amount of 30 - 99% by weight and comprising 1 - 70% by weight of one or more of man-made polyester fibre material, polyethylene fibre material, polypropylene fibre material, polyamide fibre material or elastane fibre material,
- an aqueous solution of hydrochloric acid, the aqueous solution having a concentration of hydrochloric acid of 30% - 45% by weight,
- an organic extraction solvent, and wherein the organic extraction solvent is chosen such that at 20°C less than 1 g of extraction solvent dissolves in 100 ml of the aqueous HCI solution, and wherein the organic extraction solvent is further chosen such that at 20°C at least 10 g of 5-(chloromethyl)furfural can be dissolved in 100 ml of the organic extraction solvent,
b. mixing the contents of the reactor at a temperature of between 40°C and 110°C for at least 30 minutes and wherein the aqueous solution and the organic extraction solvent are present in the reactor in a ratio aqueous solution : organic extraction solvent of between 1 : 0.5 and 1: 10,
c. separating the organic extraction solvent containing 5-(chloromethyl)furfural from the aqueous solution comprising hydrochloric acid.

2. Process for treating mixed fabric material, said mixed fabric material comprising cellulosic fibre material in an amount of 30 - 99% by weight and comprising 1 - 70% by weight of one or more of man-made polyester fibre material, polyethylene fibre material, polypropylene fibre material, polyamide fibre material, or elastane fibre material, said process comprising:
a. providing in a reactor:
- said mixed fabric material, comprising cellulosic fibre material in an amount of 30 - 99% by weight and comprising 1 - 70% by weight of one or more of man-made polyester fibre material, polyethylene fibre material, polypropylene fibre material, polyamide fibre material, or elastane fibre material,
- an aqueous solution of hydrochloric acid, the aqueous solution having a concentration of hydrochloric acid of 36% - 51% by weight,
for a time of at least 30 minutes and at a temperature of below 40°C to at least partly hydrolyse the cellulose in the cellulose fibre material, to yield an aqueous hydrolysate and solid fibrous residue,
b. removing from the product of a. the solid fibrous residue,
c. mixing the aqueous hydrolysate obtained by b. with an organic extraction solvent at a temperature of between 50°C and 110°C for at least 30 minutes and wherein the aqueous solution and the organic extraction solvent are present in the reactor in a ratio aqueous solution : organic extraction solvent of between 1 : 0.5 and 1: 10, and wherein the organic extraction solvent is chosen such that at 20°C less than 1g of extraction solvent dissolves in 100 ml of the aqueous HCI solution and wherein the organic extraction solvent is further chosen such that at 20°C at least 10 g of 5-(chloromethyl)furfural can be dissolved in 100 ml of the organic extraction solvent,
d. separating the organic extraction solvent containing 5-(chloromethyl)furfural from the aqueous solution comprising hydrochloric acid.

3. Process according to claim 1, wherein after stage b. any solid matter, including solid fibre material residue, is removed from the liquid phase.

4. Process according to claim 1 or 3, wherein in stage b. the mixing of the contents of the reactor is conducted for a time of between 0.5 hours and 12 hours, preferably between 1 and 10 hours.

5. Process according to claim 2, wherein in stage c. the mixing of the contents of the reactor is conducted for a time of between 0.5 hours and 12 hours, preferably between 1 and 10 hours.

6. Process according to claim 2 or 3, wherein the non-liquid matter that is removed comprises one or more of polyester fibre material, polyethylene fibre material, polypropylene fibre material, polyamide fibre material, or elastane fibre material.

7. Process according to any of the preceding claims wherein the organic extraction solvent comprises diisopropyl ether, ethyl acetate, pentane, hexane, heptane, octane, decane, dodecane, cyclohexane, benzene, toluene, xylene, 1,2-dichloroethane, carbon tetrachloride, trichloromethane, halogenated aromatic solvents, and mixtures thereof.

8. Process according to claim 7, wherein the organic extraction solvent comprises toluene, halogenated aromatic solvents, 1,2-dichloroethane, or mixtures thereof.

9. Process according to claim 8, wherein the halogenated aromatic solvent comprises chlorobenzene, *o*-dichlorobenzene, *m*-dichlorobenzene, *p*-dichlorobenzene, 1,2,3-trichlorobenzene, 1,2,4-trichlorobenzene, 1,3,5-trichlorobenzene, fluorobenzene, *o*-difluorobenzene, *m*-difluorobenzene, *p*-difluorobenzene, 1,2,3-trifluorobenzene, 1,2,4-trifluorobenzene, 1,3,5-trifluorobenzene, mixed halobenzene compounds, and mixtures thereof.

10. Process according to any of the preceding claims, wherein the cellulosic fibre comprises cotton.

11. Process according to any of the preceding claims, wherein the amount of mixed fabric material :
aqueous solution of hydrochloric acid in a. is between 1 : 200 and 1 : 5, preferably between 1 : 100 and 1 : 20.

12. Process according to any of the preceding claims, wherein the mixed fabric material comprises cellulosic fibre material in an amount of 40 - 98% by weight, preferably 50-98% by weight.

13. Process according to any of the preceding claims, wherein the mixed fabric material comprises 2 - 40% by weight of one or more of man-made polyester fibre material, polyethylene fibre material, polypropylene fibre material, preferably 2 -30% by weight, and wherein the man-made polyester is preferably PET.

14. Process according to any of the preceding claims, wherein the 5-(chloromethyl)furfural obtained in solution in the last stage claimed is isolated from the organic extraction solvent.

15. Process according to any of the preceding claims, wherein the mixed fabric material comprises woven material, non-woven material, braided material or mixtures thereof.

## Patentansprüche

1. Ein Verfahren zur Behandlung von Mischgewebematerial, wobei das Mischgewebematerial 30 - 99 Gew.-% Cellulosefaser-Material und 1 - 70 Gew.-% eines oder mehrerer Materialien aus synthetischem Polyesterfaser-Material, Polyethylenfaser-Material, Polypropylenfaser-Material, Polyamidfaser-Material oder Elasthan-Faser-Material umfasst, **dadurch gekennzeichnet, dass** das Verfahren umfasst:
a. Bereitstellen in einem Reaktor von:
- dem genannten Mischgewebematerial, enthaltend 30 - 99 Gew.-% Cellulosefaser-Material und 1 - 70 Gew.-% eines oder mehrerer Materialien aus synthetischem Polyesterfaser-Material, Polyethylenfaser-Material, Polypropylenfaser-Material, Polyamidfaser-Material oder Elasthan-Faser-Material,
- einer wässrigen Salzsäurelösung mit einer Konzentration von 30 - 45 Gew.-% Salzsäure,
- einem organischen Extraktionsmittel, wobei das organische Extraktionsmittel derart ausgewählt ist, dass sich bei 20°C weniger als 1 g des Extraktionsmittels in 100 ml der wässrigen HCI-Lösung löst, und wobei das organische Extraktionsmittel ferner derart ausgewählt ist, dass sich bei 20°C mindestens 10 g von 5-(Chlormethyl)furfural in 100 ml des organischen Extraktionsmittels lösen,
b. Mischen des Reaktorinhaltes bei einer Temperatur von 40°C bis 110°C über wenigstens 30 Minuten, wobei das Verhältnis wässrige Lösung : organisches Extraktionsmittel im Reaktor zwischen 1 : 0,5 und 1 : 10 liegt,
c. Abtrennen des organischen Extraktionsmittels, enthaltend 5-(Chlormethyl)furfural, von der wässrigen Lösung umfassend Salzsäure.

2. Ein Verfahren zur Behandlung von Mischgewebematerial, wobei das Mischgewebematerial 30 - 99 Gew.-% Cellulosefaser-Material und 1 - 70 Gew.-% eines oder mehrerer Materialien aus synthetischem Polyesterfaser-Material, Polyethylenfaser-Material, Polypropylenfaser-Material, Polyamidfaser-Material oder Elasthan-Faser-Material umfasst, **dadurch gekennzeichnet, dass** das Verfahren umfasst:
a. das Bereitstellen in einem Reaktor von:
- dem genannten Mischgewebematerial, umfassend 30 - 99 Gew.-% Cellulosefaser-Material und 1 - 70 Gew.-% eines oder mehrerer Materialien aus synthetischem Polyesterfaser-Material, Polyethylenfaser-Material, Polypropylenfaser-Material, Polyamidfaser-Material oder Elasthan-Faser-Material,
- einer wässrigen Salzsäurelösung mit einer Konzentration von 36 - 51 Gew.-% Salzsäure,
für eine Zeitdauer von mindestens 30 Minuten und bei einer Temperatur unter 40°C, um die Cellulose im Cellulosefaser-Material wenigstens teilweise zu hydrolysieren und ein wässriges Hydrolysat sowie einen festen faserigen Rückstand zu erhalten,
b. das Entfernen des festen faserigen Rückstandes aus dem Produkt von a.,
c. das Mischen des durch b. erhaltenen wässrigen Hydrolysats mit einem organischen Extraktionsmittel bei einer Temperatur von 50°C bis 110°C über mindestens 30 Minuten, wobei das Verhältnis wässrige Lösung : organisches Extraktionsmittel im Reaktor zwischen 1 : 0,5 und 1 : 10 liegt, und wobei das organische Extraktionsmittel derart ausgewählt ist, dass sich bei 20°C weniger als 1 g des Extraktionsmittels in 100 ml der wässrigen HCI-Lösung löst, und wobei das organische Extraktionsmittel ferner derart ausgewählt ist, dass sich bei 20°C mindestens 10 g von 5-(Chlormethyl)furfural in 100 ml des organischen Extraktionsmittels lösen,
d. das Abtrennen des organischen Extraktionsmittels, enthaltend 5-(Chlormethyl)furfural, von der wässrigen Lösung umfassend Salzsäure.

3. Ein Verfahren nach Anspruch 1, wobei nach Schritt b. alle Feststoffe einschließlich faseriger Rückstände aus der Flüssigphase entfernt werden.

4. Ein Verfahren nach Anspruch 1 oder 3, wobei in Schritt b. das Mischen des Reaktorinhaltes über eine Zeitdauer von 0,5 bis 12 Stunden, vorzugsweise zwischen 1 und 10 Stunden durchgeführt wird.

5. Ein Verfahren nach Anspruch 2, wobei in Schritt c. das Mischen des Reaktorinhaltes über eine Zeitdauer von 0,5 bis 12 Stunden, vorzugsweise zwischen 1 und 10 Stunden durchgeführt wird.

6. Ein Verfahren nach Anspruch 2 oder 3, wobei das entfernte Nicht-Flüssigmaterial ein oder mehrere Materialien aus Polyesterfaser-Material, Polyethylenfaser-Material, Polypropylenfaser-Material, Polyamidfaser-Material oder Elasthan-Faser-Material umfasst.

7. Ein Verfahren nach einem der vorstehenden Ansprüche, wobei das organische Extraktionsmittel ausgewählt ist aus Diisopropylether, Ethylacetat, Pentan, Hexan, Heptan, Oktan, Dekan, Dodekan, Cyclohexan, Benzol, Toluol, Xylol, 1,2-Dichlorethan, Tetrachlorkohlenstoff, Chloroform, halogenierte aromatische Lösungsmittel und Mischungen derselben.

8. Ein Verfahren nach Anspruch 7, wobei das organische Extraktionsmittel Toluol, halogenierte aromatische Lösungsmittel, 1,2-Dichlorethan oder Mischungen derselben umfasst.

9. Ein Verfahren nach Anspruch 8, wobei das halogenierte aromatische Lösungsmittel ausgewählt ist aus Chlorbenzol, o-Dichlorbenzol, m-Dichlorbenzol, p-Dichlorbenzol, 1,2,3-Trichlorbenzol, 1,2,4-Trichlorbenzol, 1,3,5-Trichlorbenzol, Fluorbenzol, o-Difluorbenzol, m-Difluorbenzol, p-Difluorbenzol, 1,2,3-Trifluorbenzol, 1,2,4-Trifluorbenzol, 1,3,5-Trifluorbenzol, gemischten Halobenzol-Verbindungen und Mischungen derselben.

10. Ein Verfahren nach einem der vorstehenden Ansprüche, wobei die Cellulosefaser Baumwolle umfasst.

11. Ein Verfahren nach einem der vorstehenden Ansprüche, wobei das Mengenverhältnis Mischgewebematerial : wässrige Salzsäurelösung in a. zwischen 1 : 200 und 1 : 5, vorzugsweise zwischen 1 : 100 und 1 : 20 liegt.

12. Ein Verfahren nach einem der vorstehenden Ansprüche, wobei das Mischgewebematerial 40 - 98 Gew.-% Cellulosefaser-Material, vorzugsweise 50 - 98 Gew.-% umfasst.

13. Ein Verfahren nach einem der vorstehenden Ansprüche, wobei das Mischgewebematerial 2 - 40 Gew.-% eines oder mehrerer Materialien aus synthetischem Polyesterfaser-Material, Polyethylenfaser-Material, Polypropylenfaser-Material, vorzugsweise 2 - 30 Gew.-%, umfasst, wobei das synthetische Polyester vorzugsweise PET ist.

14. Ein Verfahren nach einem der vorstehenden Ansprüche, wobei das in Lösung erhaltene 5-(Chlormethyl)furfural im letzten beanspruchten Schritt aus dem organischen Extraktionsmittel isoliert wird.

15. Ein Verfahren nach einem der vorstehenden Ansprüche, wobei das Mischgewebematerial Gewebe, Vlies, Geflecht oder Mischungen dessen umfasst.

## Revendications

1. Procédé de traitement d'un matériau en tissu mixte, ledit matériau en tissu mixte comprenant un matériau en fibres cellulosiques dans une proportion de 30 à 99 % en poids et comprenant de 1 à 70 % en poids d'au moins un parmi un matériau en fibres de polyester synthétiques, un matériau en fibres de polyéthylène, un matériau en fibres de polypropylène, un matériau en fibres de polyamide ou un matériau en fibres d'élasthanne, ledit procédé comprenant les étapes suivantes :
a. introduire dans un réacteur :
- ledit matériau en tissu mixte comprenant un matériau en fibre cellulosique dans une proportion de 30 à 99 % en poids et comprenant de 1 à 70 % en poids d'au moins un parmi un matériau en fibres de polyester synthétiques, un matériau en fibres de polyéthylène, un matériau en fibres de polypropylène, un matériau en fibres de polyamide ou un matériau en fibres d'élasthanne,
- une solution aqueuse d'acide chlorhydrique, la solution aqueuse ayant une concentration en poids de 30 à 45 %,
- un solvant d'extraction organique, dans lequel le solvant d'extraction organique est choisi de sorte qu'à 200 °C, moins de 1 g de solvant d'extraction se dissolve dans 100 ml de solution aqueuse de HCI, le solvant d'extraction organique étant en outre choisi de sorte qu'à 20 °C, au moins 10 g de 5-(chlorométhyl)furfural puissent être dissous dans 100 ml du solvant d'extraction organique,
b. mélanger le contenu du réacteur à une température comprise entre 40 et 110 °C pendant au moins 30 minutes, dans lequel la solution aqueuse et le solvant d'extraction organique sont présents dans le réacteur dans un rapport solution aqueuse/solvant d'extraction organique compris entre 1:0,5 et 1:10,
c. séparer le solvant d'extraction organique contenant du 5-(chlorométhyl)furfural de la solution aqueuse comprenant de l'acide chlorhydrique.

2. Procédé de traitement d'un matériau en tissu mixte, ledit matériau en tissu mixte comprenant un matériau en fibres cellulosiques dans une proportion de 30 à 99 % en poids et comprenant de 1 à 70 % en poids d'au moins un parmi un matériau en fibres de polyester synthétiques, un matériau en fibres de polyéthylène, un matériau en fibres de polypropylène, un matériau en fibres de polyamide ou un matériau en fibres d'élasthanne, ledit procédé comprenant les étapes suivantes :
a. introduire dans un réacteur :
- ledit matériau en tissu mixte comprenant un matériau en fibres cellulosiques dans une proportion de 30 à 99 % en poids et comprenant de 1 à 70 % en poids d'au moins un parmi un matériau en fibres de polyester synthétiques, un matériau en fibres de polyéthylène, un matériau en fibres de polypropylène, un matériau en fibres de polyamide ou un matériau en fibres d'élasthanne.
- une solution aqueuse d'acide chlorhydrique, la solution aqueuse ayant une concentration d'acide chlorhydrique de 36 à 51 % en poids
pendant au moins 30 minutes et à une température inférieure à 40 °C pour hydrolyser au moins partiellement la cellulose dans le matériau en fibres de cellulose, afin d'obtenir un hydrolysat aqueux et un résidu fibreux solide.
b. éliminer le résidu fibreux solide du produit de l'étape a,
c. mélanger l'hydrolysat aqueux obtenu à l'étape b avec un solvant d'extraction organique à une température comprise entre 50 et 110 °C pendant au moins 30 minutes, dans lequel la solution aqueuse et le solvant d'extraction organique sont présents dans le réacteur dans un rapport solution aqueuse/solvant d'extraction organique compris entre 1:0,5 et 1:10, et le solvant d'extraction organique est choisi de sorte qu'à 200 °C, moins de 1 g de solvant d'extraction se dissolve dans 100 ml de la solution aqueuse d'HCI, le solvant d'extraction organique étant en outre choisi de sorte qu'à 20 °C, au moins 10 g de 5-(chlorométhyl)furfural puissent être dissous dans 100 ml du solvant d'extraction organique.
d. séparer le solvant d'extraction organique contenant du 5-(chlorométhyl)furfural de la solution aqueuse contenant de l'acide chlorhydrique.

3. Procédé selon la revendication 1, dans lequel, après l'étape b, toute matière solide, y compris le résidu de matériau fibreux solide, est retirée de la phase liquide.

4. Procédé selon la revendication 1 ou 3, dans lequel, à l'étape b, le contenu du réacteur est mélangé pendant une durée comprise entre 0,5 et 12 heures, de préférence entre 1 et 10 heures.

5. Procédé selon la revendication 2, dans lequel, à l'étape c, le contenu du réacteur est mélangé pendant une durée comprise entre 0,5 et 12 heures, de préférence entre 1 et 10 heures.

6. Procédé selon la revendication 2 ou 3, dans lequel la matière non liquide retirée comprend au moins un parmi un matériau en fibres de polyester, un matériau en fibres de polyéthylène, un matériau en fibres de polypropylène, un matériau en fibres de polyamide ou un matériau en fibres d'élasthanne.

7. Procédé selon l'une quelconque des revendications précédentes, dans lequel le solvant d'extraction organique comprend de l'éther diisopropylique, de l'acétate d'éthyle, du pentane, de l'hexane, de l'heptane, de l'octane, du décane, du dodécane, du cyclohexane, du benzène, du toluène, du xylène, du 1,2-dichloroéthane, du tétrachlorure de carbone, du trichlorométhane, des solvants aromatiques halogénés et des mélanges de ceux-ci.

8. Procédé selon la revendication 7, dans lequel le solvant d'extraction organique comprend du toluène, des solvants aromatiques halogénés, du 1,2-dichloroéthane, ou des mélanges de ceux-ci.

9. Procédé selon la revendication 8, dans lequel le solvant aromatique halogéné comprend du chlorobenzène, de l'o-dichlorobenzène, du m-dichlorobenzène, du p-dichlorobenzène, du 1,2,3-trichlorobenzène, du 1,2,4trichlorobenzène, du 1,3,5-trichlorobenzène, du fluorobenzène, de l'o-difluorobenzène, du m-difluorobenzène, du 10 p-difluorobenzène, du 1,2,3-trifluorobenzène, du 1,2,4-trifluorobenzène, du 1,3,5-trifluorobenzène, des composés mixtes d'halobenzène, et des mélanges de ceux-ci.

10. Procédé selon l'une quelconque des revendications précédentes, dans lequel la fibre cellulosique comprend du coton.

11. Procédé selon l'une quelconque des revendications précédentes, dans lequel la proportion matériau en tissu mixte/solution aqueuse d'acide chlorhydrique à l'étape a est comprise entre 1:200 et 1:5, de préférence entre 1:100 et 1:20.

12. Procédé selon l'une quelconque des revendications précédentes, dans lequel le matériau en tissu mixte comprend un matériau en fibres cellulosiques dans une proportion de 40 à 98 % en poids, de préférence de 50 à 98 % en poids.

13. Procédé selon l'une quelconque des revendications précédentes, dans lequel le matériau en tissu mixte comprend 2 à 40 % en poids d'au moins un parmi un matériau en fibres de polyester synthétiques, un matériau en fibres de polyéthylène, un matériau en fibres de polypropylène, de préférence 2 à 30 % en poids, et dans lequel le polyester synthétique est de préférence du PET.

14. Procédé selon l'une quelconque des revendications précédentes, dans lequel le 5-(chlorométhyl)furfural obtenu en solution à l'étape finale revendiquée est isolé du solvant d'extraction organique.

15. Procédé selon l'une quelconque des revendications précédentes, dans lequel le matériau en tissu mixte comprend un matériau tissé, un matériau non tissé, un matériau tressé ou des mélanges de ceux-ci.
